# EUROPÄISCHE PATENTSCHRIFT

(11) **EP 2 412 448 B1**
(45) Veröffentlichungstag und Bekanntmachung des Hinweises auf die Patenterteilung: **06.09.2017**
(21) Anmeldenummer: 11174826.5
(22) Anmeldetag: 21.07.2011
(51) Int. Cl.: B05C 17/005, A61C 5/62, A61M 5/315

(54) **Kapsel und Kolben**
Capsule and piston
Capsule et piston

(30) Priorität: 30.07.2010 DE 102010036782
(43) Veröffentlichungstag der Anmeldung: 01.02.2012
(73) Patentinhaber: Transcodent GmbH & Co. KG, 24149 Kiel (DE)
(72) Erfinder: Spreizer, Heinrich, 8272 Ermatingen (CH)
(74) Vertreter: Lermer, Christoph

(56) Entgegenhaltungen:
- DE-A1- 3 148 490
- DE-A1- 4 232 062
- DE-A1- 19 700 480
- US-A1- 2003 196 914

## Beschreibung

### TECHNISCHES GEBIET

Die vorliegende Erfindung betrifft eine Kapsel zur Aufnahme und Dosierung hochviskoser Materialien, beispielsweise von Dentalmaterialien, mit einem Gehäuse, wobei das Gehäuse einen Innenraum begrenzt und eine Öffnung zum Einbringen eines Kolbens und eine Ausbringöffnung aufweist; und wobei das Gehäuse folgende Abschnitte umfasst: einen ersten Gehäuseabschnitt, der einen ersten zylindrischen Innenraumabschnitt begrenzt, wobei der erste Gehäuseabschnitt eine erste zentrale Längsachse, ein erstes Ende und ein zweites Ende aufweist; einen zweiten Gehäuseabschnitt, der eine Austrittsdüse bildet, wobei der zweite Gehäuseabschnitt eine zweite zentrale Längsachse, ein erstes Ende und ein zweites Ende aufweist, und der zweite Gehäuseabschnitt einen Austrittskanal begrenzt, der in die am zweiten Ende ausgebildete Austrittsöffnung mündet; und einen Übergangsabschnitt, dessen erstes Ende mit dem zweiten Ende des ersten Gehäuseabschnitts verbunden ist, und dessen zweites Ende mit dem ersten Ende des zweiten Gehäuseabschnitts verbunden ist; wobei sich die erste zentrale Längsachse und die zweite zentrale Längsachse in einem Winkel α schneiden. Außerdem betrifft die Erfindung einen Kolben, insbesondere zum Ausdrücken von Material aus der genannten Kapsel.

### STAND DER TECHNIK

In verschiedenen Anwendungsgebieten werden hochviskose Materialien eingesetzt, bspw. in der Dentaltechnik. Die Verbundstoffe/ Composite weisen einen hohen Füllanteil und somit hohe Standfestigkeit und Modellierbarkeit auf.

Die genannten Materialien werden in Einheiten, bspw. in Mengen zwischen 2 g bis 5 g, abgefüllt. Kleinere Mengen, wie etwa 250 mg bis 350mg, werden in standardisierten Einmalverpackungen aus Kunststoff, bspw. in Kapseln oder Tips, abgefüllt.

Je nach Composit können in der Anwendung in der Kapsel sehr hohe Auspressdrücke und an der Dosierzange sehr große Auspresskräfte entstehen. Während niedrige Auspressdrücke hohe Anwendersicherheit zur Folge haben, da die Kapseln nicht platzen können, sind für Anwender, wie z. B. Zahnärzte, ein langer und schlanker Dosierkanal von Bedeutung, um eine komfortable Anwendung zu gewährleisten. Für eine präzise Handhabung sind geringst mögliche Auspresskräfte vorteilhaft. Zwischen diesen Anforderungen ist eine optimierte Lösung zu finden.

So ist aus der US 2003/0196914 A1 ein Behälter für lichthärtende Materialien bekannt, bei dem das in einer Kammer des Behälters enthaltene Material mittels eines Kolbens über eine Düse ausgebracht wird.

Die DE 42 30 062 A1 D2 beschreibt eine Kapsel zum Mischen und Anwenden eines mit Flüssigkeit aktivierten Pulverzements. Auch hier wird der in einen Körper der Kapsel enthaltene Zement mittels eines Stopfens über eine Düse ausgebracht. Der Kapselkörper hat am Übergang zur Düse einen zerbrechlichen Verschluss.

Die DE 197 00 480 A1 offenbart eine Dentalpatrone bei der das enthaltene Dentalmaterial durch einen verschiebbaren Kolben über eine Ausgussspritze ausgetrieben wird.

Die DE 31 48 490 A1 hat einen als Ausdrückkolben für zähplastische Massen dienenden Bodenverschluss für einen hohlzylindrischen Strangpresszylinder zum Gegenstand.

### AUFGABE DER ERFINDUNG

Ausgehend davon besteht die Aufgabe der vorliegenden Erfindung darin, eine Kapsel und einen Kolben zum Ausbringen von Materialien hoher Viskosität, beispielsweise von Dentalmaterialien, bereitzustellen, wobei das Ausbringen mit verringerten Auspressdrücken und geringer Entmischung des ausgebrachten Materials bei geringen Totvolumina ermöglicht wird.

### TECHNISCHE LÖSUNG

Diese Aufgabe wird gelöst durch die Bereitstellung einer Kapsel gemäß dem Anspruch 1 und einer Kombination aus Kapsel und Kolben nach Anspruch 11. Vorteilhafte Ausführungsformen ergeben sich aus den Merkmalen der abhängigen Ansprüche.

Eine erfindungsgemäße Kapsel zur Aufnahme und Dosierung hochviskoser Materialien umfasst ein Gehäuse, wobei das Gehäuse einen Innenraum begrenzt und eine Öffnung zum Einbringen eines Kolbens und eine Ausbringöffnung aufweist; und wobei das Gehäuse folgende Abschnitte umfasst: einen ersten Gehäuseabschnitt, der einen ersten, insbesondere zylindrischen, Innenraumabschnitt begrenzt, wobei der erste Gehäuseabschnitt eine erste zentrale Längsachse, ein erstes Ende und ein zweites Ende aufweist; einen zweiten Gehäuseabschnitt, der eine Austrittsdüse bildet, wobei der zweite Gehäuseabschnitt eine zweite zentrale Längsachse, ein erstes Ende und ein zweites Ende aufweist, und der zweite Gehäuseabschnitt einen Austrittskanal begrenzt, der in die am zweiten Ende ausgebildete Austrittsöffnung mündet; und einen Übergangsabschnitt, dessen erstes Ende mit dem zweiten Ende des ersten Gehäuseabschnitts verbunden ist, und dessen zweites Ende mit dem ersten Ende des zweiten Gehäuseabschnitts verbunden ist; wobei sich die erste zentrale Längsachse und die zweite zentrale Längsachse in einem Winkel α von 40 bis 50°, vorzugsweise 45° schneiden. Der Übergangsabschnitt verjüngt sich zwischen seinem ersten Ende und seinem zweiten Ende, und der Verjüngungswinkel β beträgt wenigstens 50° bis 90°.

Durch die vorgeschlagene Geometrie werden die erforderlichen Auspressdrücke verringert, da die Geometrie des Innenraums das Fließverhalten begünstigt. Die Fließwiderstände beim Auspressen vom Dentalmaterialien werden gesenkt. Durch die geringeren Auspressdrücke können die Austrittskanäle im Durchmesser kleiner ausfallen. Das verringert Materialverlust in der Anwendung. Außerdem sinken Zeit- und Kraftaufwand beim Ausbringen der Dentalmaterialien. Die Auspresskraft konnte beispielsweise um 20-30% reduziert werden.

Als Materialien für die Kapsel kommen Kunststoffe wie PE, insbesondere PA, PBT, POM etc., in Frage.

Um Verbesserungen zu erzielen, wurde die Innenkontur der Kapseln optimiert. Die Vorteile sind insbesondere auf den relativ steilen Verjüngungswinkel β zurückzuführen. Durch den Übergang reduziert sich außerdem die Neigung der hochviskosen Materialien zur Entmischung. Offensichtlich weisen hochviskose Dentalmaterialien beim Übergang von einem großen Innendurchmesser auf einen kleinen Innendurchmesser mittels eines stumpfen Kegels geringeren Fließwiderstand auf.

Die Untergrenze für den Verjüngungswinkel, beträgt 50°. Kleinere Winkel verschlechtern die Eigenschaften der Kapsel erheblich. Bei niedrigeren Kegelwinkeln steigt der Fließwiderstand erheblich an.

Ein weiterer Vorteil der erfindungsgemäßen Geometrie besteht in der einfachen Herstellbarkeit. Ein erster Zylinderkern mit stumpfem Kegelübergang (Kegelstumpf-Übergang) und ein Düsenkern mit rechtwinkliger Kontaktfläche zum Kegelstumpf weisen bei der angegebenen Struktur keine Hinterschneidungen auf. Die Struktur ist somit mit zwei Kernen herstellbar bzw. entformbar.

Der Winkel α zwischen der ersten zentralen Längsachse und der zweiten zentralen Längsachse beträgt vorzugsweise zwischen 10° und 80°, insbesondere zwischen 30° und 60°, besonders bevorzugt zwischen 40° und 45°. Erfindungsgemäß verjüngt sich der Übergangsabschnitt in Form eines schiefen Kegelstumpfes, wobei der Verjüngungswinkel β dem Öffnungswinkel des Kegels entspricht. Bei schiefen oder schrägen Kegeln befindet sich die Spitze abseits der Lotgeraden durch den Mittelpunkt des Grundkreises. Die Verwendung eines schiefen bzw. schrägen Kegelstumpfes (mit großem Öffnungswinkel) im Übergangsbereich ist durch den Winkel α zwischen der ersten und der zweiten Achse notwendig.

Der schiefe Kegelstumpf kann gegenüber der ersten zentralen Längsachse verkippt in einem Winkel (γ) angeordnet sein, wobei der Winkel (γ) insbesondere 10° bis 20° beträgt. Dadurch kann ein Kolben tiefer in den Übergangsabschnitt eintauchen, wodurch die Menge des nicht ausbringbaren Materials (Verlustmaterial) verringert wird.

Insbesondere verbindet der Übergangsabschnitt den ersten Inneraumabschnitt mit dem Austrittskanal, wobei die Übergänge zwischen dem ersten Inneraumabschnitt und dem Innenraum des Übergangsabschnitts bzw. dem Innenraum des Übergangsabschnitts und dem Austrittskanal stetig sein können. In einer weiteren bevorzugten Ausführungsform verbindet der Übergangsabschnitt den ersten Inneraumabschnitt mit dem Austrittskanal, wobei der Übergang zwischen dem Übergangsabschnitt und dem Austrittskanal ein Wandstück aufweist, das quer zur Strömungsrichtung des Füllmaterials angeordnet ist. Auf diese Weise wird eine Strömungskante gebildet.

Insbesondere kann das Wandstück ringförmig ausgebildet und senkrecht zur zweiten zentralen Längsachse angeordnet sein.

Der Durchmesser (d) des Innenraums des Übergangsabschnitts nimmt vorzugsweise vom ersten Ende zur äußeren Abgrenzung des Wandstücks hin stetig und mit konstanter Steigung ab.

Der zweite Gehäuseabschnitt kann sich über das Wandstück an den Übergangsabschnitt anschließen.

Der Übergang zwischen dem Übergangsabschnitt und dem Austrittskanal kann eine Strömungskante aufweisen. Beim Passieren des Wandabschnitts bzw. an der Strömungskante findet eine mikroskopische Veränderung des Materials statt. Durch die Scherbelastung werden die Molekülketten teilweise "entschlauft" bzw.entflochten und in die Fließrichtung orientiert, d. h. ihre Vernetzung wird gelöst. Dadurch verändert sich die Viskosität in der Grenzschicht und bewirkt ein positives Fließverhalten in der Düse. Nachdem der Fließdruck abgebaut wird verkflechten, verknäulen bzw. verschlaufen sich die Makromoleküle zurück. Eine Entmischung des Füllmaterials nach dem Austritt aus der Düse findet nicht statt. Mit Hilfe der Strömungskante 35 wird eine weitere deutlich messbare Reduzierung und Beruhigung der Auspresskräfte erreicht. Das Fließverhalten ist stetig und kontinuierlich ohne stärkere Variationen der Auspresskraft.

Der Durchmesser (d1) des ersten Inneraumabschnitts ist insbesondere größer als der Durchmesser (d2) des Austrittskanals. Außen im Bereich des ersten Endes des ersten Gehäuseabschnitts kann ein Flansch ausgebildet sein. Dieser ist in der Regel ringförmig.

Vorzugsweise nimmt der Durchmesser (d) des Innenraums des Übergangsabschnitts vom ersten Ende zum zweiten Ende hin stetig und mit konstanter Steigung ab. Die Steigung kann aus der Verjüngung des Übergangsbereichs berechnet werden.

In einer bevorzugten Ausführungsform der Erfindung schließt sich der Übergangsabschnitt unmittelbar an den ersten Gehäuseabschnitt an, und der zweite Gehäuseabschnitt schließt sich unmittelbar oder über das bereits erwähnte quer stehende Wandstück an den Übergangsabschnitt an.

Die Gehäusewand kann im Bereich des der Übergangsabschnitts verstärkt sein. Für das Auspressen der Materialien aus der Kapsel gibt es auf dem Markt standardisierte Auspresswerkzeuge. Dadurch sind die Außenkontur am Kragen und die Bohrung weitgehend festgelegt. Da immer härtere Materialien ausgebracht werden sollen, werden die Werkzeuge mit immer größeren Übersetzungen angeboten. Dadurch steigt beim Auspressen die Belastung der Kapsel. Typischerweise platzt dabei eine herkömmliche Kapsel im Übergangsbereich zur Düse. Hier entstehen auch die höchsten Kräfte und der Querschnitt ist durch den Austrittskanal geschwächt. Bei der Optimierung des Bodens wurde errechnet, dass eine Verstärkung um ca. 30% bis 50% die Festigkeit ausreichend erhöht. Die Verstärkung ist an der Mantelfläche des Übergangsbereichs vorgesehen.

Vorzugsweise beträgt der Verjüngungswinkel β zwischen 50° und 120°, insbesondere zwischen 50° und 70°. Stumpfere Winkel wirken sich weniger negativ aus als spitzere Winkel.

Der Austrittskanal ist vorzugsweise zylindrisch ausgebildet. In einer anderen Ausführungsform kann der Austrittskanal als Konus ausgebildet sein, der sich mit einem Winkel von 1° bis 3° zur Düsenaustrittsöffnung hin öffnet.

Der erste Durchmesser (d1) ist insbesondere wenigstens doppelt so groß ist wie der zweite Durchmesser (d2). So kann das Verhältnis d1/d2 beispielsweise im Bereich von 2,0-4,0 liegen.

Eine weitere erfindungsgemäße Kapsel zur Aufnahme und Dosierung hochviskoser Materialien umfasst ein Gehäuse, wobei das Gehäuse einen Innenraum begrenzt und eine Öffnung zum Einbringen eines Kolbens und eine Ausbringöffnung aufweist; und wobei das Gehäuse folgende Abschnitte umfasst: einen ersten Gehäuseabschnitt, der einen ersten, insbesondere zylindrischen, Innenraumabschnitt begrenzt, wobei der erste Gehäuseabschnitt eine erste zentrale Längsachse, ein erstes Ende und ein zweites Ende aufweist; einen zweiten Gehäuseabschnitt, der eine Austrittsdüse bildet, wobei der zweite Gehäuseabschnitt eine zweite zentrale Längsachse, ein erstes Ende und ein zweites Ende aufweist, und der zweite Gehäuseabschnitt einen Austrittskanal begrenzt, der in die am zweiten Ende ausgebildete Austrittsöffnung mündet; und einen Übergangsabschnitt, dessen erstes Ende mit dem zweiten Ende des ersten Gehäuseabschnitts verbunden ist, und dessen zweites Ende mit dem ersten Ende des zweiten Gehäuseabschnitts verbunden ist; wobei sich die erste zentrale Längsachse und die zweite zentrale Längsachse in einem Winkel α von 40 bis 50°, vorzugsweise 45° schneiden. Der Übergang zwischen dem Übergangsabschnitt und dem Austrittskanal weist ein Wandstück auf, das quer zur Strömungsrichtung des Füllmaterials angeordnet ist. Auf diese Weise wird eine Strömungskante gebildet.

Die Aufgabe wird auch gelöst durch die Bereitstellung eines erfindungsgemäßen Kolbens, der eine dem Austrittskanal zugewandte Stirnseite und eine Abstreifkante bzw. -lippe aufweist, wobei eine Übergangsfläche der Stirnfläche, die an die Abstreifkante angrenzt, mit der Längsachse des Kolbens einen Winkel (δ) von 90° bis 120° einschließt, wobei der Winkel (δ) derart angeordnet ist, dass in der bestimmungsgemäßen Schubrichtung zum Ausbringen des Füllmaterials die Übergangsfläche nach außen hin schräg nach hinten verläuft. Der Begriff "nach außen hin" bezieht sich auf eine Richtung von der Längsachse des Kolbens zu den Innenwänden der Kapsel hin, der Begriff "nach hinten" bedeutet eine Richtung entgegengesetzt zur bestimmungsgemäßen Schubrichtung des Kolbens relativ zur Kapsel zum Ausbringen des Materials. Mit Hilfe des Kolbens wird das Füllmaterial während des Ausbringvorgangs von der Kapselinnenwand abgeschabt, ohne mit dieser zu verkanten.

Der Winkel (δ) beträgt insbesondere zwischen 105° und 120°.

An der Abstreifkante bzw. -lippe ist insbesondere wenigstens eine Erhöhung (Vorsprung oder Rippe, die sich vorzugsweise in Längsrichtung des Kolbens erstreckt) ausgebildet.

Der Kolben kann aus einem teilelastischen Werkstoff hergestellt sein, der sich bei hohem Druck an die Zylinderwandung anpresst. Durch den Druck wird der Kolben so verformt, dass eine für das auszubringende Material ausreichende Dichtigkeit und Stabilität zwischen der Kontaktfläche und der Zylinderinnenwand hergestellt wird. Die Kontaktfläche des Kolbens kann auch beschichtet sein, um die Gleitfähigkeit zu erhöhen.

In einer besonders bevorzugten Ausführungsform ist der Kolben aus einem relativ steifen und/oder harten Kunststoff hergestellt, um ein Verkanten bzw. Verkeilen mit der Innenwand der Kapsel zu verhindern.

Der Kolben kann wenigstens eine Abstreiflippe aufweisen, wobei an der Abstreiflippe wenigstens eine Erhöhung ausgebildet ist. Die Erhöhung ist am Außenumfang der Abstreiflippe angeordnet und schafft einen kleinen Zwischenraum zur Entlüftung zwischen der Innenwand der Kapsel und der Abstreiflippe.

Der Kolben kann symmetrisch sein und zwei Dicht- und Abstreiflippen haben. Die Lippen haben am Umfang jeweils drei Erhöhungen. Dadurch kann zunächst die Luft zwischen Composit und Kolben nach hinten entweichen. Bei Auspressen des Dentalmaterials ist die Kapsel luftfrei.

Die Aufgabe wird insbesondere auch gelöst durch eine Kombination aus der erfindungsgemäßer Kapsel und dem erfindungsgemäßen Kolben.

Dabei kann insbesondere zwischen der Abstreifkante und der Innenwand des ersten Abschnitts ein Spalt, beispielsweise von 20 µm bis 50 µm Breite, angeordnet sein. Auf diese Weise ist keine Vorspannung zwischen der Kante 511a bzw. 511b und der Innenwand des Kolbens vorhanden. Zudem kann Luft durch den Spalt entweichen (Entlüftung).

Die Bildung toter Ecken und Volumina wird bei einer Kombination aus der erfindungsgemäßer Kapsel und dem erfindungsgemäßen Kolben vermieden, da das Volumen des Übergangsbereichs durch den großen Verjüngungswinkel relativ gering ist. Die Restmenge an Material in der Kapsel nach dem Ausbringen (die sich aus dem Restmaterial in den drei Bereichen der Kapsel zusammensetzt) ist als Verlustmenge zu betrachten. Da die auszubringenden Materialien sehr teuer sein können, wird die möglichst geringe Restmenge angestrebt. Der Kolben erfüllt dazu zwei Funktionen, nämlich ein Abstreifen des Dentalmaterials von der Wand und ein möglichst tiefes Eintauchen in den Kegel. Da bei der vorliegenden Kapsel das Volumen des Übergangsbereichs wegen des stumpfen Verjüngungswinkels gering ist, ist auch die Restmenge des darin verbleibenden Materials gering. Die Restmenge wird somit durch die Restmenge in der Düse bestimmt, die durch Düsenlänge und den Düsendurchmesser vorgegeben ist.

Es muss zudem im Rahmen der Erfindung kein kegelartiger Kolben verwendet werden, um das Material möglichst vollständig aus der Kapsel auszubringen. Die Verwendung kegelartiger Kolben mit kegelförmiger Stirnfläche führt nämlich dazu, dass sich Material zwischen der Innenwand der Kapsel und dem Kolben sammeln und dort verkleben kann. Die Kombination aus erfindungsgemäßer Kapsel und erfindungsgemäßem Kolben ist daher auch in Bezug auf die Vermeidung von zusätzlicher Reibung beim Ausbringen vorteilhaft.

Insgesamt wird ein geringerer Widerstand zwischen Kolben und Zylinder erreicht. Zu den in der Kapsel beim Dosieren entstehenden sehr hohen Drücken bzw. Fließkräften addiert sich der Widerstand zwischen Kolben und Kapsel. Beim Auspressen des Materials muss der gesamte Widerstand überwunden werden. Dieser kann bei ungünstigen Formen sehr hoch sein. Übliche Kolben mit steiler, kegelartiger Stirnfläche können sehr hohe Reibkräfte bewirken, da sich das Material zwischen dem Kolbenkegel und der Zylinderwand verkeilt. Dabei kann es zu einer Entmischung kommen. Dies reduziert zum einen die Qualität des Produktes, zum anderen verklebt der Kolben in der Kapsel.

Wie beschrieben werden die Nachteile durch die erfindungsgemäße Kombination überwunden. Die Stirnfläche des Kolbens bildet im Kontaktbereich zur Kapselinnenwand einen rechten Winkel oder einen negativen Winkel, beispielsweise von -10° bis -30°. Dies bedeutet, dass sich zwischen der Kapselwand und dem Kolben ein spitzer Winkel kleiner als 90° ausbildet. Der Randbereich der Kolbenstirnwand verläuft, bezogen auf die Ausbringrichtung, nach hinten schräg. Dadurch wird das Material sauber von der Wand abgestreift. Es muss lediglich die Gleitreibung zwischen Kolben und Zylinder überwunden werden. Klebeeffekte oder Entmischung treten nicht auf. Der Widerstand kann durch die Verwendung geeigneter Materialien für Kapsel und Kolben und/oder durch eine Beschichtung des Kolbens, wenigstens im Kontaktbereich, optimiert werden. Als Beschichtung kommen beispielsweise Teflondispersion oder eine Teflonbeimischung in das Kolbenmaterial in Frage.

Schutz soll auch für ein Herstellungsverfahren zur Herstellung einer Kapsel und/oder eines Kolbens wie oben beschrieben beansprucht werden. Die Teile werden in einem Spritzgussverfahren gefertigt, wobei bei der Kapselherstellung zwei Kerne verwendet werden, nämlich ein erster Kern für die zylindrische Austrittsdüse und ein zweiter Kern für den ersten Innenraumabschnitt und den Kegelstumpfartigen Übergangsbereich. Die Machbarkeit wird dadurch sichergestellt, dass keine Hinterspritzung zwischen den zwei Kernen vorhanden ist. Die Kerne stoßen ohne Hinterschnitt im rechten Winken aufeinander. Querkräfte treten nicht auf. Eine Toleranzverschiebung wird dadurch zugelassen, dass im Übergangsbereich der Durchmesser des Kegelschnitts (zweites Ende des Übergangsbereichs) um einen vorbestimmten Betrag (z. B. 0,2 mm bis 0,5 mm) größer gefertigt wird als der Durchmesser der Austrittsdüse.

Für die beschriebenen Merkmale soll in allen möglichen neuen Kombinationen Schutz beansprucht werden.

### KURZE BESCHREIBUNG DER FIGUREN

Weitere Merkmale und Vorteile der Erfindung werden aus der nachfolgenden Beschreibung eines bevorzugten Ausführungsbeispiels anhand der beigefügten Figuren deutlich. Die Figuren zeigen:
Figur 1 eine Draufsicht auf eine erste Ausführungsform einer erfindungsgemäßen Kapsel;
Figur 2 eine erste Schnittansicht der erfindungsgemäßen Kapsel aus Figur 1;
Figur 3 eine zweite Schnittansicht der erfindungsgemäßen Kapsel aus Figur 1;
Figur 4 einen perspektivische Ansicht einer ersten Ausführungsform eines erfindungsgemäßen Kolbens;
Figur 5 eine Draufsicht auf die Stirnfläche einer zweiten Ausführungsform eines erfindungsgemäßen Kolbens;
Figur 6 eine erste Schnittansicht einer zweite Ausführungsform einer erfindungsgemäßen Kapsel;
Figur 7 eine zweite Schnittansicht der erfindungsgemäßen Kapsel aus Figur 6;
Figur 8 ein Detail der erfindungsgemäßen Kapsel aus Figur 6;
Figur 9 einen perspektivische Längsansicht einer dritten Ausführungsform eines erfindungsgemäßen Kolbens;
Figur 10 ein Längsschnitt durch den Kolben aus Figur 9;
Figur 11 eine vordere Draufsicht auf den Kolben aus Figur 9; und
Figur 12 ein Detail aus Figur 11.

### DETAILLIERTE BESCHREIBUNG BEVORZUGTER AUSFÜHRUNGSBEISPIELE

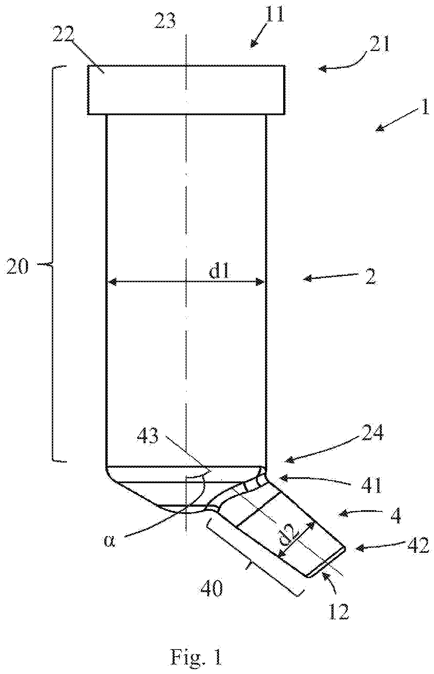

Die beigefügte Figur 1 zeigt eine erfindungsgemäße Kapsel 1 zur Aufnahme und zum Ausbringen hochviskoser Dentalmaterialien. Die Kapsel 1 ist aus Kunststoff hergestellt.

Die Kapsel weist ein Gehäuse 10 auf, das einen Innenraum begrenzt. Das Gehäuse 10 weist zum einen eine erste Öffnung 11 zum Eindrücken eines Kolbens (nicht dargestellt) auf, zum anderen eine zweite Öffnung 12, die als Ausbringöffnung zum Austritt des Dentalmaterials dient, wenn der Kolben über die erste Öffnung in den Innenraum gepresst wird.

Das Gehäuse und der Innenraum weisen einen ersten Abschnitt 2, einen Übergangsbereich 3 und einen zweiten Abschnitt 4 (Austrittsdüse) auf.

Der erste Abschnitt 2 weist einen ersten Gehäuseabschnitt 20 auf, an dessen ersten Ende 21 die erste Öffnung 11 der Kapsel 1 ausgebildet ist. Am ersten Ende des ersten Gehäuseabschnitts 20 ist außerdem ein sich nach außen erstreckender Flansch 22 ausgebildet, der insbesondere kreisförmig um den Rand der Öffnung 11 verläuft. Der Innenraum des ersten Gehäuseabschnitts 20 ist im Wesentlichen zylindrisch mit kreisförmigem Querschnitt, konstantem Durchmesser d1, und einer ersten zentralen Längsachse 23. An das zweite Ende 24 des ersten Gehäuseabschnitts 20 schließt sich unmittelbar ein erstes Ende 31 des Übergangsbereichs 3 an.

Das zweite Ende 32 des Übergangsbereichs mündet in den zweiten Abschnitt 4, der als zylindrische Austrittsdüse mit einer zweiten zentralen Längsachse 43 konzipiert ist. Der Innendurchmesser d2 des zweiten Abschnitts 4 ist konstant und kleiner als der Innendurchmesser d1 des ersten Abschnitts 2. Während sich das erste Ende 41 des Gehäuses 40 unmittelbar an den Übergangsbereich 30 anschließt, weist das zweite Ende 42 die Austrittsöffnung 12 auf.

Die erste zentrale Längsachse 23 und die zweite zentrale Längsachse 43 schließen einen Winkel α von ca. 45° ein.

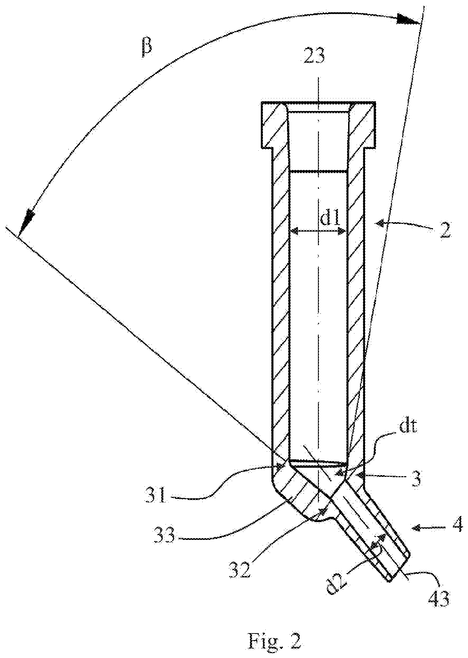

Wie aus der Figur 2 hervorgeht, weist der Innenraum des Übergangsbereichs 3 einen Innendurchmesser dt auf, der sich zu seinem zweiten Ende 32 hin stetig verjüngt bzw. verringert. Der Durchmesser dt am ersten Ende 31 entspricht dem Durchmesser d1 des ersten Gehäuseabschnitts 20. Der entlang des Übergangsbereichs 3 variierende Durchmesser dt entspricht am zweiten Ende 32 dem Innendurchmesser d2 des zweiten Gehäuseabschnitts 40. Die Verjüngung erfolgt stetig und (innerhalb des Übergangsbereichs 3) mit konstanter Steigung. Dies bedeutet, dass der Innenraum des Übergangsbereichs 3 durch die Mantelfläche eines Kegelstumpfes begrenzt wird. Der dem Kegelstumpf entsprechende Kegel ist ein schräger (schiefer) Kegel mit einem Öffnungswinkel β, der dem Verjüngungswinkel entspricht. Der Öffnungswinkel β beträgt im vorliegenden Ausführungsbeispiel ca. 55°. Damit ist die Verjüngung relativ steil, was überraschend zu geringeren Auspresskräften im Innenraum bei konstanten Ausbringzeiten und zu geringerer Entmischung des Dentalmaterials führt.

An der bei 32 angedeuteten Linie stoßen die beiden bei der Herstellung im Spritzguss verwendeten Kerne aneinander.

Eine Materialverdickung 33 im Bereich der Gehäusewand, die die erste Längsachse 23 schneidet, sorgt für die nötige Stabilität, um die hohen Belastungen während des Ausbringens des Materials aufzunehmen.

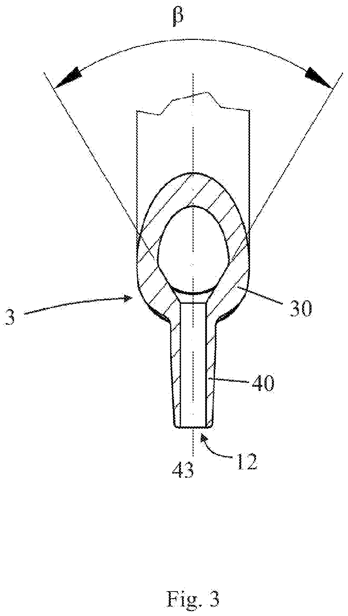

Die Figur 3 ist eine weitere Schnittdarstellung. Der Schnitt erstreckt sich durch die Austrittsdüse 2 sowie den Übergangsbereich 3. Der Innenraum des Übergangsbereichs 3 (begrenzt durch die Wand 30) mündet mit einem Verjüngungswinkel β in den durch die Wand 40 begrenzten Austrittskanal.

Mittels der Erfindung kann das Fließverhalten bei gleichzeitig reduzierter Neigung zum Entmischen verbessert werden. Dies führt dazu, dass beim Dosieren hochviskoser Materialien die Dosierkräfte gegenüber herkömmlich verwendeten Düsen erheblich gesenkt werden können, d. h. in der Anwendung sind geringere Kräfte erforderlich, wodurch die Kraft beim Dosieren verringert werden kann.

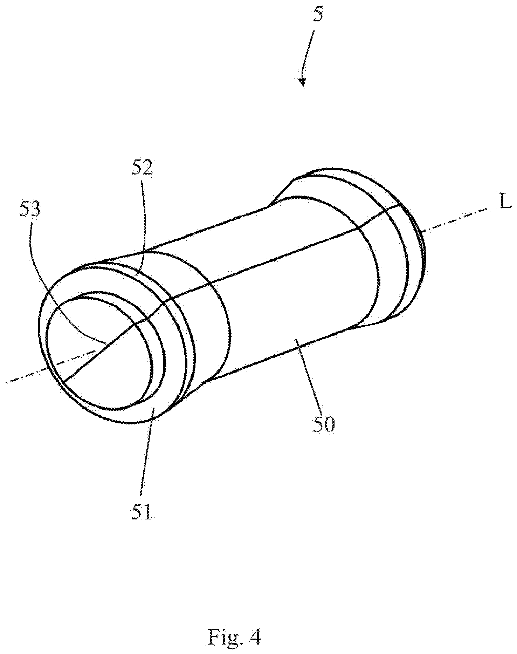

Die Figur 4 zeigt eine Ausführungsform eines erfindungsgemäßen Stößels/Kolbens 5 zum Ausbringen von Dentalmaterial beispielsweise aus einer Kapsel 1 wie oben beschrieben. Dazu wird der Stößel 5 durch die erste Öffnung 11 eingeführt und in Richtung der Austrittsdüse 4 nach unten gepresst. Durch die Volumenreduzierung entweicht zunächst die Restluft nach hinten, danach fließt das Material aus der Austrittsöffnung 12 aus.

Der Stößel 5 ist in der Regel aus Kunststoff hergestellt. Er weist einen Grundkörper 50 mit einer Stirnfläche 51 auf, die zuerst in den Innenraum der Kapsel eingeführt wird. Die Stirnfläche 51 bildet mit der Innenwand der Kapsel einen rechten Winkel bzw. die Stirnwand schließt mit der Kapselwand einen spitzen Winkel kleiner 90° ein, d. h. es ist ein negativer Winkel von -10° bis -30° vorhanden. Dadurch wird das Material sauber von der Wand abgestreift. Es muss im Wesentlichen nur die Gleitreibung zwischen dem Kolben 5 und der Innenwand des Zylinders überwunden werden. Klebeeffekte oder Entmischung werden vermieden. Der Widerstand kann durch Beschichtung des Kolbens, insbesondere im Bereich der Kontaktfläche/-kante 52, optimiert werden. Insgesamt wird der Widerstand zwischen Kolben und Zylinderinnenwand reduziert.

Beim automatischen Einbringen des Kolbens 5 durch die Kapselöffnung 11 und beim Auspressen der Materialien muss Luft aus dem eingeschlossenen Innenraum der Kapsel nach hinten entweichen können. Das auszubringende Material darf dabei nicht nach hinten in Richtung der ersten Öffnung 11 entweichen, da eine Kontamination des Stößels der Auspresszange zu vermeiden ist. Aus diesem Grund ist der Kolbenkörper 50 mit einem Entlüftungsspalt 53 ausgebildet. Dieser ist groß genug, sodass Luft sicher entweichen kann, allerdings nicht durchlässig für das auszubringende hochviskose Material.

Insgesamt ist der Kolben 5 symmetrisch weist zwei noch außen hervorstehende Dicht- und Abstreifkanten bzw. -lippen 52 auf, die geringe Reibung auf die Wände der Kapsel ausüben. Der Kolben 5 hat zwei Dicht- und Abstreiflippen.

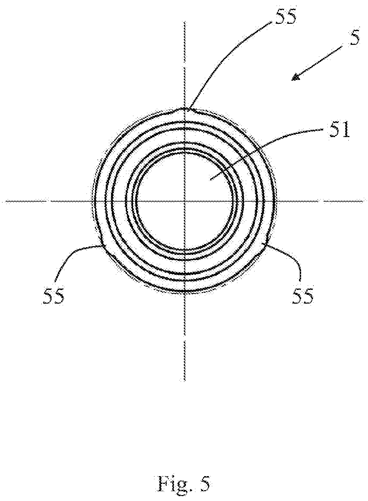

Die Figur 5 ist eine Draufsicht auf die Stirnfläche 51 eines erfindungsgemäßen Kolbens 5, der dem Kolben aus der Figur 4 gleicht. Zusätzlich weist der Kolben 5 bei dieser Ausführungsform (an beiden Dichtlippen jeweils) drei Erhöhungen 55 auf, die den Reibwiderstand zu den Kapselwänden um ca. 3% bis 5 % erhöhen. Diese Kraft ist in der Anwendung nicht spürbar, sorgt aber dafür, dass der Kolben sicher in der Kapsel gehalten wird und (insbesondere beim Verpacken) nicht verloren geht. Allerdings ist der durch die Erhöhungen entstehende Zwischenraum zwischen Kapselwand und Dichtkante so klein, dass zwar eine Entlüftung möglich ist, d. h. Luft zwischen die Dichtlippen treten kann, das Dentalmaterial jedoch nicht durch den Spalt hindurch treten kann.

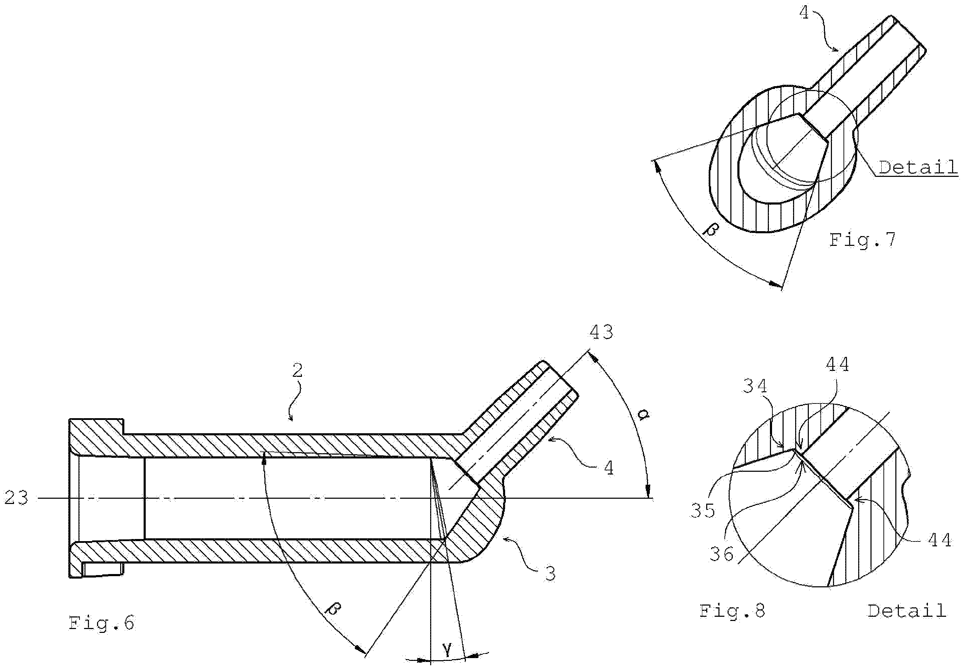

Eine weitere Reduktion der Auspresskräfte gelingt mit einer Variation der erfindungsgemäßen Kapsel gemäß den Figuren 6, 7 und 8.

Die Kapsel ist prinzipiell wie die in den Figuren 1 bis 3 dargestellte und beschriebene Kapsel aufgebaut. Entsprechende Komponenten sind daher mit denselben Bezugszeichen wie im vorherigen Ausführungsbeispiel gekennzeichnet.

Der Winkel α zwischen der ersten zentralen Längsachse 23 und der zweiten zentralen Längsachse 43 beträgt ca. 45°. Der Übergangsbereich 3 entspricht einem Kegelstumpf. Ein entsprechender schräger (schiefer) Kegel weist einen Öffnungswinkel β = 55° auf der dem Verjüngungswinkel entspricht.

Am Übergang 34 zwischen dem Übergangsbereich 3 und der Austrittsdüse 2, der in der Detaildarstellung gemäß Figur 8 besonders deutlich erkennbar ist, weist ein Wandstück 35 auf. Das Wandstück 35 ist ein in etwa ringförmiger Wandabschnitt, vorzugsweise mit einer Breite von 0,1 mm bis 0,25 mm. Das Wandstück 35 liegt senkrecht zur Flussrichtung bzw. zur Längsachse 43 des zweiten Abschnitts 4 (Austrittsdüse) und wirkt als eine Art Blende. Der ringförmige Wandabschnitt schließt sich einerseits an den kegelförmigen Bereich des Übergangsbereichs 3 und andererseits an das erste Ende 41 des Gehäuses 40 des zweiten Abschnitts 4 an. Der den ringförmige Wandabschnitt bildende Teil 44 des Gehäuses 40 bildet eine Art Vorsprung für das in die Austrittsdüse 4 fließende Füllmaterial. Die Übergangskante bzw. Strömungskante 36 zwischen dem ringförmigen Wandabschnitt 35 und dem zweiten Abschnitt 4 ist scharfkantig.

Beim Passieren des Wandabschnitts 35 bzw. an der Strömungskante 36 findet eine mikroskopische Veränderung des Materials statt. Durch die Scherbelastung werden die Molekülketten teilweise "entschlauft" und in die Fliessrichtung orientiert, d. h. ihre Vernetzung wird gelöst. Dadurch verändert sich die Viskosität in der Grenzschicht und bewirkt ein positives Fließverhalten in der Düse. Nachdem der Fließdruck abgebaut wird verknäulen und verschlaufen sich die Makromoleküle zurück. Eine Entmischung des Füllmaterials nach dem Austritt aus der Düse findet nicht statt. Mit Hilfe der Strömungskante 35 wird eine weitere deutlich messbare Reduzierung und Beruhigung der Auspresskräfte erreicht. Die Kräfte unterliegen keinen größeren Schwankungen, d. h. die Kurve ist stetig. Das Fließverhalten ist stetig und kontinuierlich ohne stärkere Variationen der Auspresskraft.

Als weitere Abwandlung gegenüber der ersten Ausführungsform besteht darin, dass der kegelförmige Abschnitt des Übergangsbereichs 3 gegenüber dem ersten Abschnitt 2 verkippt wurde. In diesem Ausführungsbeispiel ist der Kegel in Richtung der zweiten Längsachse 43 verkippt. Der Winkel γ der Verkippung beträgt hier 10°. Die Strömung wird durch die Verkippung nicht beeinflusst. Bei jeder der Ausführungsformen bleibt beim Auspressen, in Abhängigkeit von der Kolbenform und vom Kegelstumpf, Restmaterial in der Kapsel. In der vorliegenden Ausführungsform kann dieses Restmaterial reduziert werden, da durch die Verkippung der Kolben tiefer in den kegelförmigen Übergangsbereich 3 eintauchen kann. Der Materialverlust bei einem Ausbringvorgang kann so um ca. 30 % verringert werden, wobei der Materialverlust in der Düse gegenüber der ersten Ausführungsform unverändert bleibt.

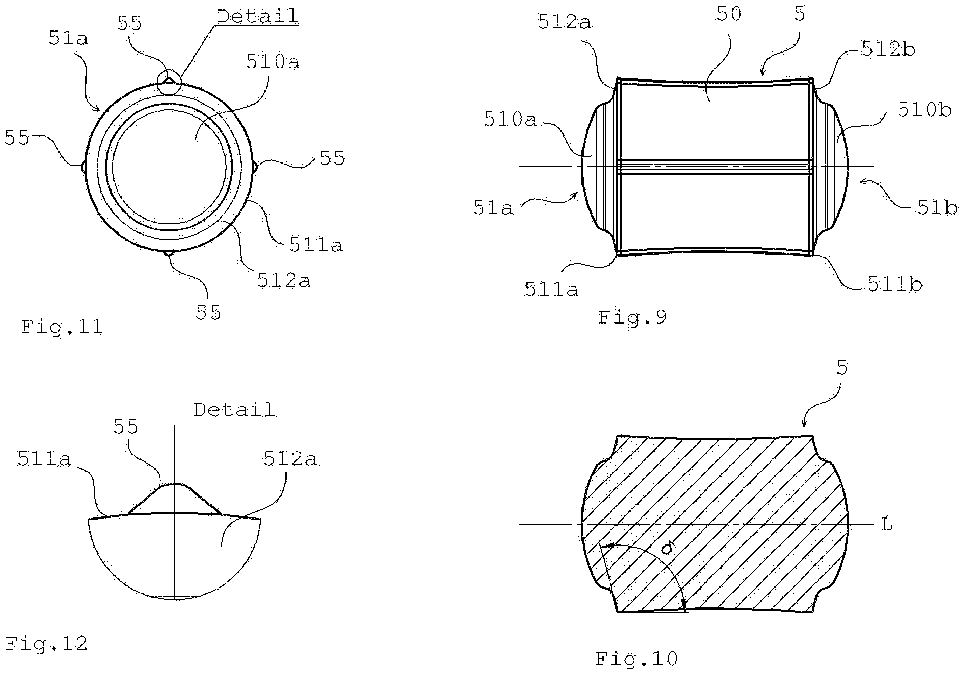

In der Figur 9 ist eine dritte Ausführungsform eines erfindungsgemäßen Kolbens 5 dargestellt. Soweit nichts anderes beschrieben ist, entspricht der Kolben 5 den vorher beschriebenen Ausführungsformen. Entsprechende Komponenten sind daher mit denselben Bezugszeichen wie in den vorherigen Ausführungsbeispielen gekennzeichnet.

Der Kolben 5 weist einen Grundkörper 50 mit einer vorderseitigen Stirnfläche 51a und einer rückseitigen Stirnfläche 51b auf. Der Kolben 5 ist hinsichtlich des vorderen und hinteren Bereichs symmetrisch aufgebaut. Die Stirnflächen 51a und 51b weisen jeweils einen konkaven Vorsprung 510a bzw. 510b auf.

Der Kolben 5 ist vorzugsweise aus einem relativ harten Kunststoff hergestellt. Im Gegensatz zu der ersten und zweiten Ausführungsform werden die Stirnflächen 51a und 51b jeweils durch eine kreisförmige Abstreifkante 511a bzw. 511b begrenzt, die einen Radius derart aufweist, dass zwischen der Innenwand der Kapsel und der Abstreifkante 511a bzw. 511b (zumindest bei unbelastetem Kolben) ein Spalt, beispielsweise von 20 µm bis 50 µm Breite, vorhanden ist. Auf diese Weise ist auch keine Vorspannung zwischen der Kante 511a bzw. 511b und der Innenwand des Kolbens vorhanden. Die Verhältnisse sind (ähnlich wie in der Grenzschicht) so, dass Auspresskräfte reduziert und ein Verklemmen verhindert werden.

Zudem kann Luft durch den Spalt entweichen (Entlüftung). Das Füllmaterial wird durch den Kolben 5 von der Innenwand der Kapsel abgeschabt.

Zwischen dem Vorsprung 510a bzw. 510b und der jeweiligen Kante 511a bzw. 511b ist eine Übergangsfläche 512a bzw. 512b angeordnet. Diese ist im Wesentlichen ringförmig, verläuft jedoch mit negativer Steigung zur Kante 511a bzw. 511b hin. D. h. die Fläche 512a bzw. 512b fällt, bei Draufsicht auf die jeweilige Stirnseite 51a bzw. 51b, zur Kante 511a bzw. 511b, nach außen hin, ab. Dies zeigt besonders deutlich die Figur 10, in der die negative Steigung der Fläche 512a eingezeichnet ist. Der Winkel d ist ein Maß für die Steigung bzw. das Gefälle der Fläche 512a nach außen hin. Der Winkel δ beträgt vorzugsweise etwa 105° bis 120°.

Aus der Figur 10 wird ein weiteres Merkmal des Kolbens 5 deutlich. Durch das Schwindverhalten der Kunststoffe kann der Spritzprozess so gestaltet werden, dass der Zylinderradius in Richtung der Längsachse L zur Mitte hin kontrolliert ca. 0,2 mm abnimmt. D. h. der Kolben weist eine in Längsrichtung konkave Zylinderoberfläche auf. Dadurch wird ein unerwünschtes Verklemmen des Kolbens 5 in der Kapsel vermieden.

Die Figuren 11 und 12 zeigen ein weiteres Merkmal der dritten Ausführungsform des Kolbens 5. Dieses Merkmal kann, muss jedoch nicht Teil der dritten Ausführungsform sein.

Am Umfang der äußeren Mantelwand des Kolbens 5 sind Vorsprünge bzw. Rippen 55 ausgebildet. Diese haben eine Überdeckung mit der Kapsel von ca. 0,1 mm bis 0,2 mm. Der Kolben wird somit, wenn er in die Kapsel eingebracht ist, in dieser festgehalten und kann nicht herausfallen bzw. herausgezogen werden. Durch die Vorsprünge werden Toleranzen sicher kompensiert und der Kolben 5 mit reproduzierbaren Kräften in der Kapsel gehalten. Der Kolben ist über die Vorsprünge 55 mit der Kapsel verspannt. Da die dadurch ausgeübten Kräfte jedoch nur ca. 3 % bis 5 % der Auspresskräfte betragen, ist diese Zusatzkraft beim Ausbringen für den Benutzer nicht wahrnehmbar. Beim Transport oder Verpacken können andererseits die Kolben nicht verloren gehen, da sie sicher in den Kapseln gehalten werden.

Es können drei (3) bis sechs (6) Rippen angebracht werden, abhängig vom Kapsel- und Kolbenwerkstoff und dem Verhalten der Polymere (Füllmaterial).

Wenn auch nicht in einer Figur dargestellt wirken Kapseln und Kolben wie oben beschrieben in unterschiedlichen Kombinationen zusammen. Das Ergebnis ist ein verbessertes Ausbringen des Füllmaterials mit geringeren Auspresskräften, höherer Zuverlässigkeit und geringeren Anforderungen an die Bauweise der Kapsel - Auspresswerkzeuge.

## Patentansprüche

1. Kapsel (1) zur Aufnahme und Dosierung hochviskoser Materialien, mit einem Gehäuse, wobei
das Gehäuse einen Innenraum begrenzt und eine Öffnung (11) zum Einbringen eines Kolbens und eine Ausbringöffnung (12) aufweist; und
wobei das Gehäuse folgende Abschnitte umfasst:
einen ersten Gehäuseabschnitt (20), der einen ersten Innenraumabschnitt begrenzt, wobei der erste Gehäuseabschnitt (20) eine erste zentrale Längsachse (23), ein erstes Ende (21) und ein zweites Ende (24) aufweist;
einen zweiten Gehäuseabschnitt (40), der eine Austrittsdüse bildet, wobei der zweite Gehäuseabschnitt (40) eine zweite zentrale Längsachse (43), ein erstes Ende (41) und ein zweites Ende (42) aufweist, und der zweite Gehäuseabschnitt (40) einen Austrittskanal begrenzt, der in die am zweiten Ende (42) ausgebildete Austrittsöffnung (12) mündet; und
einen Übergangsabschnitt (3), dessen erstes Ende (31) mit dem zweiten Ende (24) des ersten Gehäuseabschnitts (20) verbunden ist, und dessen zweites Ende (32) mit dem ersten Ende (41) des zweiten Gehäuseabschnitts (40) verbunden ist;
wobei sich die erste zentrale Längsachse (23) und die zweite zentrale Längsachse (43) in einem Winkel (α) schneiden,
**dadurch gekennzeichnet, dass**
sich der Übergangsabschnitt (3) zwischen seinem ersten Ende (31) und seinem zweiten Ende (32) in Form eines schiefen Kegelstumpfes verjüngt, wobei der Verjüngungswinkel (β) dem Öffnungswinkel des Kegels entspricht und der Verjüngungswinkel (β) wenigstens 50° beträgt.

2. Kapsel (1) nach Anspruch 1,
**dadurch gekennzeichnet, dass**
der schiefe Kegelstumpf gegenüber der ersten zentralen Längsachse (23) verkippt in einem Winkel (γ) angeordnet ist, wobei der Winkel (γ) insbesondere 10° bis 20° beträgt.

3. Kapsel (1) nach einem der vorhergehenden Ansprüche,
**dadurch gekennzeichnet, dass**
der Übergangsabschnitt (3) den ersten Innenraumabschnitt mit dem Austrittskanal verbindet, wobei der Übergang (34) zwischen dem Übergangsabschnitt (3) und dem Austrittskanal (4) ein Wandstück (35) aufweist, das quer zur Strömungsrichtung des Füllmaterials angeordnet ist.

4. Kapsel (1) nach Anspruch 3,
**dadurch gekennzeichnet, dass**
das Wandstück (35) ringförmig ausgebildet und senkrecht zur zweiten zentralen Längsachse (43) angeordnet ist.

5. Kapsel (1) nach Anspruch 3 oder 4,
**dadurch gekennzeichnet, dass**
der Durchmesser (d) des Innenraums des Übergangsabschnitts (3) vom ersten Ende (31) zur äußeren Abgrenzung des Wandstücks (35) hin stetig und mit konstanter Steigung abnimmt.

6. Kapsel (1) nach einem der Ansprüche 3 bis 5,
**dadurch gekennzeichnet, dass**
sich der zweite Gehäuseabschnitt (40) über das Wandstück (35) an den Übergangsabschnitt (3) anschließt.

7. Kapsel (1) nach einem der vorhergehenden Ansprüche,
**dadurch gekennzeichnet, dass**
der Übergang (34) zwischen dem Übergangsabschnitt (3) und dem Austrittskanal (4) eine Strömungskante (36) aufweist.

8. Kapsel (1) nach einem der vorhergehenden Ansprüche,
**dadurch gekennzeichnet, dass**
der Austrittskanal des zweiten Gehäuseabschnitts (40) konusförmig ausgebildet ist und sich mit einem Winkel, insbesondere in einem Winkel von 1° bis 3°, zur Austrittsöffnung (12) hin öffnet.

9. Kapsel (1) nach einem der vorhergehenden Ansprüche,
**dadurch gekennzeichnet, dass**
der Durchmesser (d) des Innenraums des Übergangsabschnitts (3) vom ersten Ende (31) zum zweiten Ende (32) hin stetig und mit konstanter Steigung abnimmt.

10. Kapsel (1) nach einem der vorhergehenden Ansprüche,
**dadurch gekennzeichnet, dass**
dass der Verjüngungswinkel (β) zwischen 50° und 120°, insbesondere zwischen 50° und 70°, beträgt.

11. Kombination aus einer Kapsel nach einem der Ansprüche 1 bis 10 und einem Kolben, wobei der Kolben (50) wenigstens eine Stirnseite (51) und eine Abstreifkante bzw. -lippe (52; 511a, 511b) aufweist, wobei eine Übergangsfläche (512a, 512b) der Stirnfläche (51), die an die Abstreifkante angrenzt, mit der Längsachse (L) des Kolbens (50) einen Winkel (δ) von 90° bis 120° einschließt, wobei der Winkel (δ) derart angeordnet ist, dass in der bestimmungsgemäßen Schubrichtung zum Ausbringen des Füllmaterials die Übergangsfläche (512a, 512b) nach außen hin schräg nach hinten verläuft und insbesondere zwischen der Abstreifkante (511a, 511b) und der Innenwand des ersten Abschnitts (2) ein Spalt angeordnet ist.

12. Kombination nach Anspruch 11,
**dadurch gekennzeichnet, dass**
der Winkel (δ) des Kolbens zwischen 105° und 120° beträgt.

13. Kombination nach Anspruch 11 oder 12,
**dadurch gekennzeichnet, dass**
an der Abstreifkante bzw. -lippe (52; 511a, 511b) des Kolbens wenigstens eine Erhöhung (55) ausgebildet ist.

## Claims

1. A capsule (1) for receiving and dosing high-viscosity materials comprising a housing,
wherein
the housing defines an interior chamber and has an opening (11) for inserting a piston, and a delivery opening (12); and
wherein the housing comprises following sections:
a first housing section (20), which defines a first interior chamber section, wherein the first housing section (20) has a first central longitudinal axis (23), a first end (21) and a second end (24);
a second housing section (40), which forms an exit nozzle, wherein the second housing section (40) has a second central longitudinal axis (43), a first end (41) and a second end (42),
and the second housing section (40) defines an exit channel, which opens into the delivery opening (12) formed at the second end (42); and
a transition section (3), whose first end (31) is connected to the second end (24) of the first housing section (20), and whose second end (32) is connected to the first end (41) of the second housing section (40);
wherein the first central longitudinal axis (23) and the second central longitudinal axis (43) intersect at an angle (α),
**characterised by the fact that**
the transition section (3) tapers between its first end (31) and its second end (32) in the form of an oblique truncated cone, with the taper angle (β) corresponding to the opening angle of the cone and the taper angle (β) is at least 50°.

2. The capsule (1) in accordance with claim 1,
**characterised by the fact that**
the oblique truncated cone is arranged such that it is tilted at an angle (γ) relative to the first central longitudinal axis (23), with the angle (γ) being especially 10° to 20°.

3. The capsule (1) in accordance with any of the previous claims,
**characterised by the fact that**
the transition section (3) connects the first interior chamber section to the exit channel, wherein the transition (34) between the transition section (3) and the exit channel (4) has a wall piece (35) that is arranged transversely to the flow direction of the filler material.

4. The capsule (1) in accordance with claim 3,
**characterised by the fact that**
the wall piece (35) is of an annular design and arranged perpendicularly to the second central longitudinal axis (43).

5. The capsule (1) in accordance with claim 4 or 5,
**characterised by the fact that**
the diameter (d) of the interior chamber of the transition section (3) decreases steadily from the first end (31) to the outer boundary of the wall piece (35) and at a constant slope.

6. The capsule (1) in accordance with any of claims 3 to 5,
**characterised by the fact that**
the second housing section (40) connects to the transition section (3) via the wall piece (35).

7. The capsule (1) in accordance with any of the previous claims,
**characterised by the fact that**
the transition (34) between the transition section (3) and the exit channel (4) can have a flow edge (36).

8. The capsule (1) in accordance with any of the previous claims,
**characterised by the fact that**
the exit channel of the second housing section (40) is formed like a cone and opens at an angle, especially an angle of 1° to 3°, to the exit opening (12).

9. The capsule (1) in accordance with any of the previous claims,
**characterised by the fact that**
the diameter (d) of the interior chamber of the transition section (3) decreases steadily from the first end (31) to the second end (32) and at a constant slope.

10. The capsule (1) in accordance with any of the previous claims,
**characterised by the fact that**
the taper angle (β) is between 50° and 120°, especially between 50° and 70°.

11. A combination of a capsule in accordance with any of claims 1 to 10, and a piston,
wherein the piston (50) has at least one end face (51) and a scraper edge or lip (52; 511a, 511b), wherein one transition surface (512a, 512b) of the end face (51), which is contiguous with the scraper edge, forms an angle (δ) of 90° to 120° with the longitudinal axis (L) of the piston (50), wherein the angle (δ) is arranged such that, in the intended thrust direction for delivery of the filler material, the outward transition surface (512a, 512b) runs obliquely rearwards and a gap is arranged especially between the scraper edge (511a, 511b) and the inner wall of the first section (2).

12. The piston (50) in accordance with claim 11,
**characterised by the fact that**
the angle (δ) is between 105° and 120°.

13. The piston (50) in accordance with claim 11 or 12,
**characterised by the fact that**
at the scraper edge or lip (52; 511a, 511b) is formed at least one elevation (55).

## Revendications

1. Capsule (1) destinée à la réception et au dosage de matières à viscosité élevée, comprenant un boîtier, dans laquelle
le boîtier limite un espace intérieur et comporte un orifice (11) pour introduire un piston ainsi qu'un orifice de distribution (12) ; et
dans laquelle le boîtier comprend les portions suivantes :
une première portion de boîtier (20), qui limite une première portion d'espace intérieur, la première portion de boîtier (20) comportant un premier axe longitudinal central (23), une première extrémité (21) et une seconde extrémité (24) ;
une seconde portion de boîtier (40), qui forme une buse de sortie, la seconde portion de boîtier (40) comportant un second axe longitudinal central (43), une première extrémité (41) et une seconde extrémité (42), et la seconde portion de boîtier (40) limitant un canal de sortie, qui débouche dans l'orifice de sortie (12) formé à la seconde extrémité (42) ; et
une portion de transition (3), dont la première extrémité (31) est reliée à la seconde extrémité (24) de la première portion de boîtier (20), et dont la seconde extrémité (32) est reliée à la première extrémité (41) de la seconde portion de boîtier (40) ;
le premier axe longitudinal central (23) et le second axe longitudinal central (43) se coupant selon un angle (α),
**caractérisée en ce que**
la portion de transition (3) se rétrécit entre sa première extrémité (31) et sa seconde extrémité (32) sous la forme d'un cône tronqué oblique, l'angle de rétrécissement (β) correspondant à l'angle d'ouverture du cône et l'ange de rétrécissement (β) étant d'au moins 50°.

2. Capsule (1) selon la revendication 1,
**caractérisée en ce que**
le cône tronqué oblique est disposé incliné selon un angle (γ) par rapport au premier axe longitudinal central (23), l'angle (γ) étant en particulier compris entre 10° et 20°.

3. Capsule (1) selon l'une des revendications précédentes,
**caractérisée en ce que**
la portion de transition (3) relie la première portion d'espace intérieur au canal de sortie, la transition (34) entre la portion de transition (3) et le canal de sortie (4) comportant une partie de paroi (35), qui est disposée transversalement au sens d'écoulement de la matière de remplissage.

4. Capsule (1) selon la revendication 3,
**caractérisée en ce que**
la partie de paroi (35) présente une forme annulaire et est disposée perpendiculairement au second axe longitudinal central (43).

5. Capsule (1) selon la revendication 3 ou 4,
**caractérisée en ce que**
le diamètre (d) de l'espace intérieur de la portion de transition (3) diminue en continu et avec une inclinaison constante de la première extrémité (31) à la limitation extérieure de la partie de paroi (35).

6. Capsule (1) selon l'une des revendications 3 à 5,
**caractérisée en ce que**
la seconde portion de boîtier (40) est contiguë à la portion de transition (3) par le biais de la partie de paroi (35).

7. Capsule (1) selon l'une des revendications précédentes,
**caractérisée en ce que**
la transition (34) entre la portion de transition (3) et le canal de sortie (4) comporte un rebord d'écoulement (36).

8. Capsule (1) selon l'une des revendications précédentes,
**caractérisée en ce que**
le canal de sortie de la seconde portion de boîtier (40) présente une forme conique et s'ouvre vers l'orifice de sortie (12) selon un angle, en particulier un angle de 1° à 3°.

9. Capsule (1) selon l'une des revendications précédentes,
**caractérisée en ce que**
le diamètre (d) de l'espace intérieur de la portion de transition (3) diminue en continu et avec une inclinaison constante de la première extrémité (31) à la seconde extrémité (32).

10. Capsule (1) selon l'une des revendications précédentes,
**caractérisée en ce que**
l'angle de rétrécissement (β) est compris entre 50° et 120°, en particulier entre 50° et 70°.

11. Combinaison d'une capsule selon l'une des revendications 1 à 10 et d'un piston, le piston (50) comportant au moins une face frontale (51) et une arête ou lèvre de raclage (52 ; 511a, 511b), une surface de transition (512a, 512b) de la face frontale (51), qui est adjacente à l'arête de raclage, formant avec l'axe longitudinal (L) du piston (50) un angle (δ) de 90° à 120°, l'angle (δ) étant disposé de telle manière que dans le sens de la poussée en fonctionnement normal pour distribuer la matière de remplissage, la surface de transition (512a, 512b) s'étend obliquement vers l'arrière vers l'extérieur et un interstice étant en particulier disposé entre l'arête de raclage (511a, 511b) et la paroi intérieure de la première portion (2).

12. Combinaison selon la revendication 11,
**caractérisée en ce que**
l'angle (δ) du piston est compris entre 105° et 120°.

13. Combinaison selon la revendication 11 ou 12,
**caractérisée en ce que**
au moins une bosse (55) est formée sur l'arête ou la lèvre de raclage (52 ; 511a, 511b) du piston.
